# EUROPEAN PATENT APPLICATION

(11) **EP 0 721 014 A1**
(43) Date of publication of application: **10.07.1996**
(21) Application number: 94925617.6
(22) Date of filing: 02.09.1994
(51) Int. Cl.: C12P 21/08, C12N 5/20, C07K 16/28

(54) **MONOCLONAL ANTIBODY THAT RECOGNIZES INTERSTITIAL CELL SURFACE ANTIGEN**

(30) Priority: 03.09.1993 JP 242109/93
(71) Applicant: CHUGAI SEIYAKU KABUSHIKI KAISHA, Tokyo 115 (JP)
(72) Inventor: FUKUSHIMA, Naoshi, Gotemba-shi, Shizuoka 412 (JP)
(74) Representative: Baillie, Iain Cameron
(86) International application number: JP9401452
(87) International publication number: WO9506747

(57) **Abstract**

It is the objective and purpose of the present invention to provide a novel and specific monoclonal antibody.

This invention relates to a monoclonal antibody inhibiting the homing of hematopoietic stem cells and recognizing the surface antigen of a splenic stromal cell having potency supportive of the homing of hematopoietic stem cells and a hybridoma producing the monoclonal antibody.

Since the monoclonal antibody of this invention has a characteristic of inhibiting the homing of hematopoietic stem cells, it is useful as medicine enhancing the effect of an anticancer agent for the clinical treatment of leukemia.

## Description

### Technical Field

The present invention relates to a novel monoclonal antibody inhibiting the homing of hematopoietic stem cells and recognizing a splenic stromal cell having potency supportive of the homing of hematopoietic stem cells, and furthermore, relates to a hybridoma producing the monoclonal antibody.

Since the monoclonal antibodies of the present invention recognize a substance important for the homing of bone marrow cells on the hematopoietic tissue as an antigen and have a characteristic of inhibiting the homing of hematopoietic stem cells, it is useful as medicine with a function of enhancing the effect of an anticancer agent, for example, improving the effect of the anticancer agent for leukemia by releasing acute myeloid leukemic cells from the bone marrow according to the above characteristic.

### Background Art

Granulocyte colony-stimulating factors, for example, recombinant granulocyte colony-stimulating factors (rG-CSF), have been known primarily as humoral factors to stimulate the differentiation and proliferation of granulocyte cells, and it has been reported in an experiment upon mice in vivo that the administration of rG-CSF enhances the hematopoiesis of the bone marrow and in addition causes remarkable extramedullary hematopoiesis in the spleen to proliferate hematopoietic stem cells and all hematopoietic precursor cells in the spleen. And it has been thought as extramedullary hematopoietic mechanism in the spleen that hematopoiesis occurs due to a splenic hematopoietic microenvironment modifications according to the stimulation of rG-CSF to enhance hematopoietic potential.

Hence, the present inventors have noted splenic stromal cells administered rG-CSF with a view to clarifying the hematopoietic potential in the spleen, and established a hematopoietic stromal cell line (CF-1 cells) from the spleen of a mouse administered rG-CSF with a view to attempting the analysis of the enhancement of the hematopoietic potential by stromal cells with rG-CSF, and examined the potential effect on hematopoiesis using the hematopoietic stromal cells, and as a result, colony-stimulating activities in vitro and potency supportive of hematopoietic stem cells in vivo have been recognized [Blood, 80, 1914 (1992)] .

Since the potency supportive of hematopoietic stem cells in vivo is recognized when the CF-1 cells are transplanted together with bone marrow cells on bone marrow transplantation, it has been thought that the above hematopoietic stromal cell line (CF-1 cells) expresses a function as a cell adhesion factor allowing hematopoietic stem cells to home on the hematopoietic tissue.

However, though some of splenic stromal cells having potency supportive of the homing of hematopoietic stem cells have been established as a cell line (CF-1 cells), and cytological characteristics thereof have been examined, no specific antibody recognizing surface antigens thereof has been prepared so far, and characteristics thereof have been scarcely known yet.

Hence, the present inventors have engaged in assiduous studies with a view to developing a specific antibody capable of recognizing a splenic stromal cell having potency supportive of the homing of hematopoietic stem cells on the basis of the above information upon splenic stromal cells and the results of the studies, and established a splenic stromal cell having potency supportive of the homing of hematopoietic stem cells as a cell line, and at the same time prepared a monoclonal antibody using the splenic stromal cell line as an antigen for immunization, and as a result, a novel monoclonal antibody has been obtained.

And the inventors have found that the obtained monoclonal antibody recognizes the surface antigen of a splenic stromal cell having potency supportive of the homing of hematopoietic stem cells and has a characteristic of inhibiting the homing of hematopoietic stem cells, which has led to the completion of the present invention.

### Disclosure of Invention

It is the objective and purpose of the present invention to provide a novel monoclonal antibody recognizing the surface antigen of a splenic stromal cell having potency supportive of the homing of hematopoietic stem cells and having a characteristic of inhibiting the homing of hematopoietic stem cells. Furthermore, it is another objective of this invention to provide a hybridoma producing the monoclonal antibody.

The monoclonal antibody of the present invention relates to a novel monoclonal antibody to the surface antigen of splenic stromal cells having potency supportive of the homing of hematopoietic stem cells, and that is an antibody recognizing a splenic stromal cell related to the enhancement of the hematopoietic potential of the spleen, and it is extremely useful as a substance with the function of inhibiting the homing of hematopoietic stem cells. Incidentally, the homing of hematopoietic stem cells here means that the adherence of the hematopoietic stem cells are caused by stromal cells of hematopoietic tissue, and the cells differentiate and proliferate as CFU-S colonies.

The monoclonal antibody of the present invention may be prepared basically as stated below.

Namely, the monoclonal antibody of this invention may be prepared, for example, by using splenic stromal cells derived from an animal administered rG-CSF, such as CF-1 cells (splenic stromal cells) established by the present inventors as a cell line, as an antigen, immunizing it according to an ordinary immunization method, cell-fusing the immunized cells according to an ordinary cell fusion method, and cloning the fused cells according to an ordinary cloning method.

As a method of preparing the monoclonal antibody of the present invention can be preferably exemplified a method comprising using the above CF-1 cells established as culture cell line by the present inventors, as the antigen [Blood, Vol. 80, 1914 (1992) ] , fusing plasma cells (immunocyte) of a mammal immunized with the antigen with myeloma cells of a mammal such as a mouse, cloning the obtained fused cells (hybridomas), selecting clones producing an antibody according to the present invention recognizing the above cell line among them, and culturing them to recover objective antibodies. However, the method is only an example, and in this case, for example, not only the above CF-1 cells but also other stromal cells of hematopoietic tissue obtained according to the case of CF-1 cells, or stromal cells of hematopoietic tissue derived from human splenic stromal cells may be used properly to produce objective antibodies in the same manner as in the case of the above CF-1 cells.

In the method of preparing such monoclonal antibodies, mammals to be immunized with the above antigen are not particularly restricted; it is preferable to make selection taking into account suitability with myeloma cells to be used in cell fusion, and preferably a mouse, a rat and a hamster are used.

Immunization is performed according to an ordinary method, for example, by administering splenic stromal cells such as the above CF-1 cells into abdominal cavity of a mammal by injection. More specifically, it is preferable to administer one diluted with or suspended in a proper amount of PBS or isotonic sodium chloride solution to an animal several times every month. It is preferable to use splenic cells removed after the final administration of the above cells as immunocytes.

As a myeloma cell of a mammal as the other parent cells fused with the above immunocytes can be used preferably known various cells including P3(P3X63Ag8.653) [J. Immunol., 123, 1548 (1978) ] , p3-U1 [Current Topics in Micro-biology and Immunology, 81, 1-7 (1978)] , NS-1 [Eur. J. Immunol., 6, 511-519 (1976) ] , MPC-11 [Cell, 8, 405-415 (1976)] , Sp2/0 - Ag14 [Nature, 276, 269-270 (1978)] , FO [J. Immunol. Meth., 35, 1-21 (1980) ] , S194 [J. Exp. Med., 148, 313-323 (1978)] and R210 [Nature, 277, 131-133 (1979)] .

The cell fusion of the above immunocyte and a myeloma cell may be performed basically according to an ordinary method, for example, a method by Milstein et al. [Methods Enzymol., 73, 3-46 (1981)] .

More specifically, the above cell fusion may be performed, for example, in an ordinary nutrition medium in the presence of a fusion-accelerating agent. As a fusion-accelerating agent, polyethylene glycol (PEG) and Sendai virus (HVJ), and furthermore, adjuvants such as dimethyl sulfoxide may be added properly if required in order to enhance the fusing effect. Regarding the ratios of immunocytes and myeloma cells, the former is preferably used in an amount 1-10 times that of the latter. Examples of a medium used in the above cell fusion include a RPMI-1640 medium and a MEM medium suitable for the proliferation of the above myeloma cell and other mediums ordinarily used for the culture of this kind of cell, and in addition, supplementary serum such as fetal bovine serum (FBS) may be used together.

Cell fusion is performed by mixing prescribed amounts of the above immunocytes and myeloma cells in the above medium, adding a PEG solution preheated to about 37°C , for example, PEG with an average molecular weight of the order of 1,000-6,000 to the medium, ordinarily, at a concentration of about 30-60 % (W/V), and mixing them. Subsequently, by repeating the operations of adding proper mediums one after another, centrifuging the reaction mixture and removing the supernatants can be formed objective hybridomas.

Said hybridomas are selected by culturing in an ordinary selective medium, for example, a HAT medium (medium supplemented with hypoxanthine, aminopterin and thymidine). The culture in the HAT medium is continued for a period sufficient for cells other than objective hybridomas (non-fused cells) to die out, ordinarily for several days to several weeks. Subsequently, the screening and monocloning of the hybridomas producing the objective antibodies are performed according to ordinary limiting dilution method.

The established hybridomas producing the monoclonal antibodies of the present invention may be subcultured in an ordinary medium and stored in liquid nitrogen for a long time.

In order to collect the monoclonal antibodies of the present invention from the hybridomas may be employed a method comprising culturing the hybridomas according to an ordinary method, and obtaining them from the supernatants, or a method comprising administering a hybridoma into a appropriate mammal to proliferate, and obtaining them from its ascite. The former is suitable for obtaining antibodies with a high purity and the latter is suitable for the mass production of antibodies.

Furthermore, the antibodies obtained according to the above method may be purified to a high degree employing an ordinary purification means such as a salting-out technique, gel filtration and affinity chromatography.

Since the monoclonal antibodies of the present invention have a potential of inhibiting the homing of hematopoietic stem cells, they can be used as medicine with a function of enhancing the effect of an anticancer agent in the clinical treatment of leukemia; for example, by utilizing the function, acute myeloid leukemic cells are released from the bone marrow to improve the effect of the anticancer agent for leukemia.

Among remedies for leukemia with the utilization of the monoclonal antibodies of the present invention, it goes without saying that the establishment of a specific system for the use thereof as medicine for improving the effect of an anticancer agent for leukemia, modification and application thereof are included within the scope of the present invention so far as they are put into practice according to an ordinary method obvious to those skilled in the art.

### Brief Description of Drawings

Fig. 1 shows an analysis (a control in the absence of an antibody, CF-1 cells) according to immunofluorescence.

Fig. 2 shows an analysis of the binding properties of the GSPST-1 antibody to CF-1 cells according to immunofluorescence.

Fig. 3 shows an analysis of the binding properties of the HMS-1 antibody to CF-1 cells according to immunofluorescence.

Fig. 4 shows an analysis (a control in the absence of an antibody, bone marrow cells) according to immunofluorescence.

Fig. 5 shows an analysis of the binding properties of the GSPST-1 antibody to bone marrow cells according to immunofluorescence.

Fig. 6 shows an analysis of the binding properties of the HMS-1 antibody to bone marrow cells according to immunofluorescence.

Fig. 7 shows an assay for the monoclonal antibodies (GSPST-1) of the present invention to inhibit the bone marrow transplantation.

Fig. 8 shows an assay for the monoclonal antibodies (HMS-1) of the present invention to inhibit the bone marrow transplantation.

Next, the present invention will be described further in detail according to Reference Example and Example, but the present invention is not restricted to the Example.

### Reference Example

### Establishment of Splenic Stromal Cells and Their Characteristics Thereof

### 1) Establishment of Splenic Stromal Cells

A splenic stromal cell line having potency supportive of hematopoietic stem cells was established from the primary culture of the splenic cells of a C57BL/6J mouse administered rG-CSF 100 µg/kg for 5 days.

Namely, this spleen was removed after the administration of rG-CSF under germ-free conditions, cultured in a 25-cm² plastic flask (Corning Co.) for 6 weeks and in an Isocove's modified Dulbecco's medium (IMDM) (Boehringer-Mannheim Co.) with 10 % heat-inactivated fetal bovine serum (FBS) (Sanko Junyaku, Tokyo), 100 U/ml penicillin and 100 µg/ml streptomycin in an incubator under the condition of 37 °C and 5 % CO₂, and the medium was exchanged for a fresh growth medium twice a week.

In the confluent culture, the adherent cell populations (stromal cells) were harvested from the flask by using 0.05 % trypsin plus 0.02 % EDTA (Sigma Chemical Co.) in Ca-,Mg-free PBS, and were transferred into new flasks. These passages were repeated approximately once or twice a week. In the early passages (1st through 10th passages), the split ratio of the cells was 1/4 to 1/8, and subsequently the ratio was 1/16 to 1/32. The stromal cells became homogeneous and fibroblastoid after approximately the 10th passage.

At the 20th passage, these stromal cells were harvested as described above and forwarded to cell cloning by using a limiting dilution technique; cell cloning was repeated twice to establish a stromal cell line (CF-1 cell line).

Subsequently, these cells were maintained in 5 ml of IMDM supplemented with 10 % heat-inactivated FBS in a 25-cm² flask (Corning Co.), and subcultured once every 5 days at the split ratio of 1/32. Splenic stromal cell lines can be established from other animals than mouse; for example, human splenic stromal cell lines can be established using the same method as described above by transforming the cells with an SV-40 adenovirus vector [J. Cell. Physiol., 148, 245 (1991) ] .

### 2) Characteristics of CF-1 Cells

CF-1 cells established as a cell line as described above were examined for alkaline phosphatase, acid phosphatase, β -glucuronidase, α -naphthyl acetate esterase and oil red O using standard cytochemical techniques. CF-1 cells were also characterized by immunoenzymatic histochemistry using the following monoclonal and polyclonal antibodies: macI (Sero Tec.); factor VIII-related antigen (Dakopatts); and collagen type I, collagen type III and fibronectin (Chemicon International Inc.). Phagocytosis was tested by latex bead uptake (particle diameter: 1.09 µm; Sigma), and the potency of CF-1 cells to convert to adipocytes was tested by exposure to 10⁻⁶ mol/l hydrocortisone phosphate (Sigma) in a 25-cm² flask for 4 weeks after the confluent culture.

As a result, the CF-1 cells were negative for alkaline phosphatase, factor VIII-related antigen, mac I and phagocytosis, whereas they were positive for collagen type I, collagen type III and fibronectin. CF-1 cells were not converted to adipocytes during 4 weeks in a confluent culture with 10⁻⁶ mol/l hydrocortisone, although CF-1 cells had only traces of lipid. From these data, CF-1 cells do not have the characteristics of preadipocytes, macrophages and endothelial cells, and therefore it has become obvious that they are derived from stromal cells different from them.

### 3) Maintenance of Hematopoietic Stem Cells by CF-1 Cells

To examine whether hematopoietic stem cells are maintained by CF-1 cells or not, CFU-S assays (assay of spleen colonies-forming cells) were performed by the technique of Till and McCulloch. Ten mice per group were irradiated with 900 cGy (MBR-1520R; Hitachi, Tokyo) and injected intravenously with bone marrow mononuclear cells (BM cells) (1.0 × 10⁵/head, 5.0 × 10⁴/head, or 2.5 × 10⁴/head) and CF-1 cells (1.0 × 10⁵/head), and colonies in the spleen were counted on the 12th day as CFU-S colonies (spleen colonies).

As a result, when bone marrow mononuclear cells (BM cells) and CF-1 cells were transplanted into irradiated mice, the number of spleen colonies of every group of BM cells increased significantly (between 1.4-1.8 times) as compared to the mice without CF-1 cells transplanted, and, on the 12th day after the transplantation, the survival ratios of the mice translanted with BM cells and CF-1 cells were higher than those with only BM cells, showing a low death rate; hence it has become apparent that hematopoietic stem cells are maintained by CF-1 cells.

### Best Mode for Carrying Out the Invention

The embodiment of the invention will be described in detail hereinafter.

### Example

### Establishment of Monoclonal Antibodies

### 1) Antigens and Immunization

Immunization was performed by using CF-1 cells obtained in the above Reference Example as antigens. The cells were subcultured in an incubator under the condition of 5 % CO₂ and 37°C , using an Isocove's modified Dulbecco's medium (IMDM) (Boehringer-Mannheim Co.) supplemented with 10 % fetal bovine serum (FBS; Sanko Junyaku) as a medium.

The cells were treated with 1mM EDTA/PBS, and removed from a culture flask according to pipetting. The cells were suspended into 1mM EDTA/PBS at the cell number of about 1 × 10⁷/ml, and administered to a Wistar Imamich rat (7-week-old, female; Animal Breeding Research Laboratory). One ml of cells of about 1× 10⁷/ml were injected into the abdominal cavity of the rat at the initial immunization, and 1 ml of cells of about 1 × 10⁷/ml were administered additionally one month later. Further, 1 ml of cells of about 1 × 10⁷/ml were administered additionally several times at an interval of a month, and after the reactivity between the immunized rat antibody and CF-1 cells was recognized, 1 ml of cells of 1 × 10⁸/ml were administered as the final immunization. Three days after the final immunization, the rat was killed to remove spleen.

### 2) Cell Fusion

After the spleen removed from the rat was minced, splenic cells isolated were centrifuged, suspended in an IMDM medium (Boehringer-Mannheim Co.), and washed intensively. On the other hand, the cells obtained by cultured mouse myeloma cell line Sp2/0-Ag14 [Nature, 276, 269-270 (1978)] in an IMDM (Boehringer-Mannheim Co.) supplemented with 10 % fetal bovine serum (FBS; Sanko Junyaku) were washed in the above IMDM medium in the same manner, and 1× 10⁸ thereof and 2 × 10⁸ of the above splenic cells were put into a centrifuge tube and mixed to perform cell fusion by polyethylene glycol 4000 (Nakarai Kagaku) according to an ordinary procedure [Clin. Exp. Immunol., 42 , 458-462 (1980)] .

Subsequently, the obtained fused cells were dispensed into a 96-well plate with an IMDM medium supplemented with 20 % FBS, and cultured in an incubator under the condition of 37 °C and 5 % CO₂. They were replaced into a HAT selective medium gradually from the following day, and continued to be cultured.

After the start of the culture, the supernatants were replaced into a new HAT medium twice a week to continue the culture and maintain the proliferation.

Next, the obtained fused cells were cloned according to an ordinary procedure using limiting dilution method. Namely, only clones having strong binding properties to antigens were cloned according to an ordinary procedure employing limiting dilution analysis by examining binding properties thereof to the antigens, utilizing antibodies in the supernatants of the above fused cells.

### 3) Screening

The screening of fused cells (hybridomas) was performed according to indirect fluorescent antibody technique using flow cytometry.

The screening of clones producing objective antibodies was performed using CF-1 cells as target cells. Namely, cells suspended in a reaction buffer (PBS suppplemented with 2 % FBS and 0.02 % NaN₃ ) were centrifuged and recovered as pellets, then suspended in 100 µl of the hybridoma culture supernatants (about 1 × 10⁶/100 µl) and reacted at 4 °C for 1 hour. After they were washed with the above buffer once, an FITC-labelled goat anti-rat IgG (FC) antibody (Chemicon) was added thereto and incubated for 1 hour. After they were washed once, they were analyzed according to flow cytometry (FACScan, Becton Dickinson).

### 4) Purification of Antibodies

The fused cells screened in the manner of the above 3) were cultured according to an ordinary procedure, and antibodies produced in the supernatants were separated according to an ordinary procedure, and purified.

Namely, hybridomas were recovered from wells with high antibody titers to the antigens, spread in a tissue culture plastic dish (Corning Co.), cultured under the condition of 5 % CO₂ and 37 °C , proliferated, and purified according to an ordinary procedure to obtain monoclonal antibodies GSPST-1 and HMS-1.

In this case, regarding the HMS-1 antibody and the GSPST-1 antibody, obtained cells were injected into the abdominal cavity of a BALB/cAJc1-nu nude mouse (8-week-old, male, Nippon Kurea), and its ascite was recovered after 10-14 days, salted out with 33 % ammonium sulfate, and dialyzed with PBS.

Incidentally, in the above Example was described the case in which the CF-1 cells were used as antigens for immunization; however, it is possible to establish a monoclonal antibody in the same manner also in case of using other splenic stromal cells including stromal cell lines of hematopoietic tissue derived from human splenic stromal cells, and the present invention is not restricted to the above monoclonal antibodies but includes all monoclonal antibodies having the same characteristics prepared in the same manner and all hybridomas producing the monoclonal antibodies.

A hybridoma producing the monoclonal antibody HMS-1 of the present invention is a novel fused cell prepared from a Wistar Imamich rat splenic cell and a mouse myeloma cell line SP2/0-Ag14 as parent cells, and was deposited on 9 August 1993, under the name of HMS-1 (rat mouse hybridoma) with the accession number of FERM BP-4383, at National Institute of Bioscience and Human Technology, Agency of Industrial Science and Technology in Japan [address:1-3, Higashi 1-chome, Tsukuba-shi, Ibaraki 305, Japan] , international depositary authority according to Budapest Treaty on the international recognition of the deposit of microorganisms for the purpose of patent procedure.

### 5) Properties of Antibodies

### (i) Reactivity of Antibodies

### (Reactivity to CF-1 Cells)

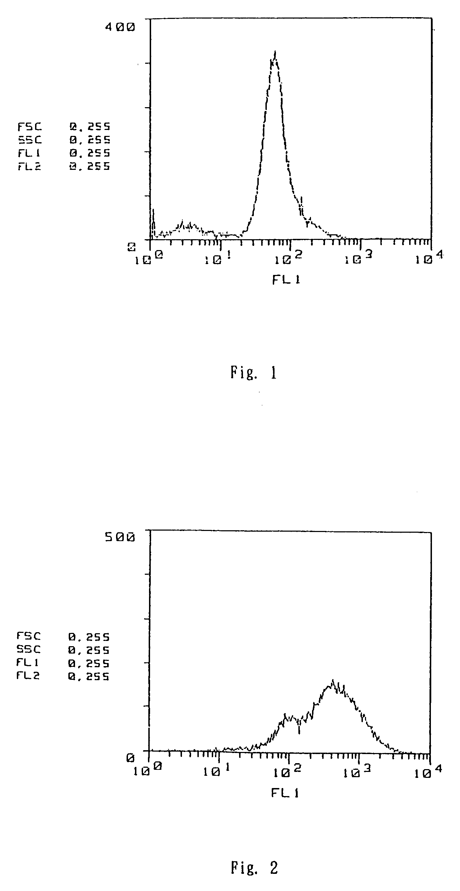

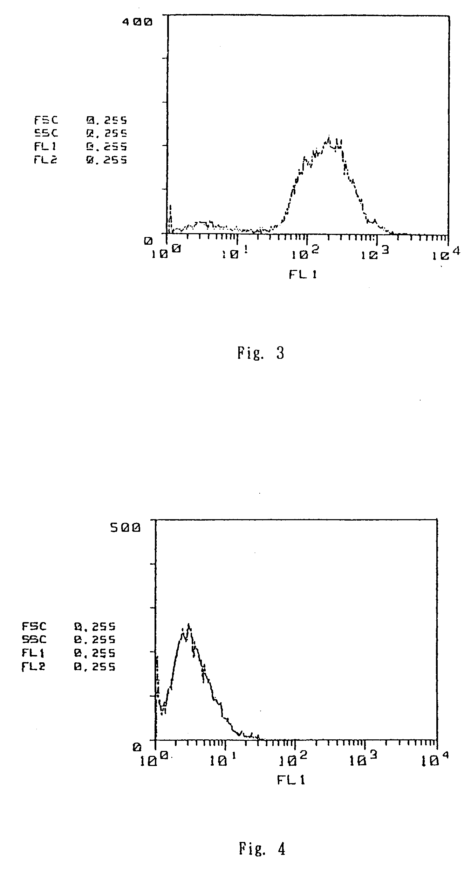

The results of examining the reactivity of the obtained monoclonal antibodies GSPST-1 and HMS-1 to CF-1 cells according to immunofluorescence analysis are shown in Fig. 1 through Fig. 3. Here, Fig. 1 shows the results of analysis of a control in the absence of an antibody, Fig. 2 the results of analysis of the binding properties of GSPST-1 to CF-1 cells, and Fig. 3 the results of analysis of the binding properties of HMS-1 to CF-1 cells. In the drawings, vertical axes show relative number of cells and transverse axes fluorescence intensity.

As is apparent from Fig. 1 through Fig. 3, it has been revealed that monoclonal antibodies GSPST-1 and HMS-1 have properties binding to CF-1 cells and recognize surface antigens of CF-1 cells.

### (Reactivity to Bone Marrow Cells)

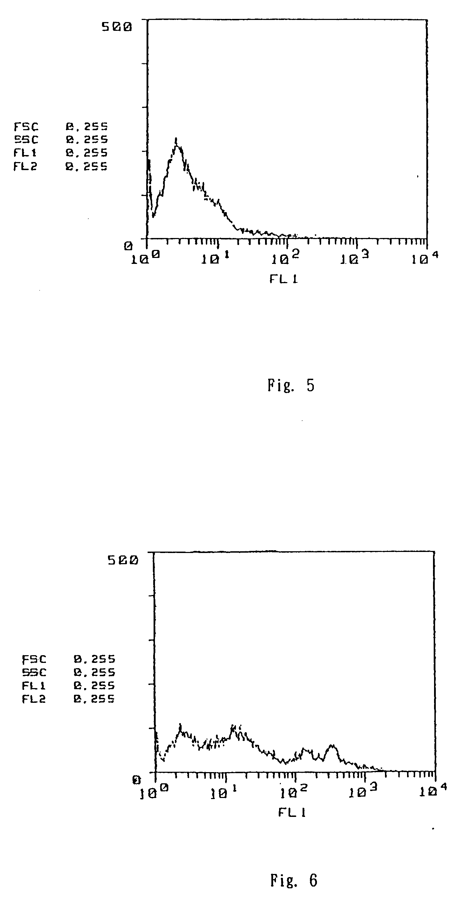

Next, the results of analysis of the reactivity of GSPST-1 and HMS-1 to normal bone marrow cells according to flow cytometry (FACScan, Becton Dickinson) are shown in Fig. 4 through Fig. 6. Here, Fig. 4 shows the results of analysis of the control in the absence of an antibody, Fig. 5 the results of analysis of the binding properties of GSPST-1 to bone marrow cells, and Fig. 6 the results of analysis of the binding properties of HMS-1 to bone marrow cells. In the drawings, vertical axes show relative number of cells and transverse axes fluorescence intensity.

As is shown in Fig. 4 through Fig. 6, it has been revealed that GSPST-1 has not a property binding to bone marrow cells at all, and that HMS-1 has a property binding to some of bone marrow cells.

### (ii) Typing of Antibodies

Next, as a result of typing the subclass of IgG of the obtained monoclonal antibodies [using rat Mono Ab-ID-Sp kit (Zymed)] , it has become apparent that GSPST-1 is IgG2a, and that HMS-1 is IgG2b.

### (iii) Potency inhibiting Bone Marrow Transplantation

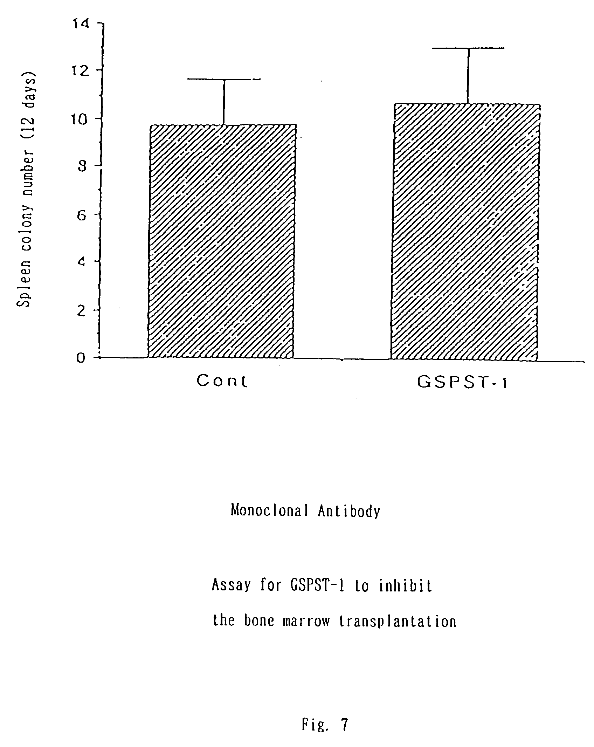

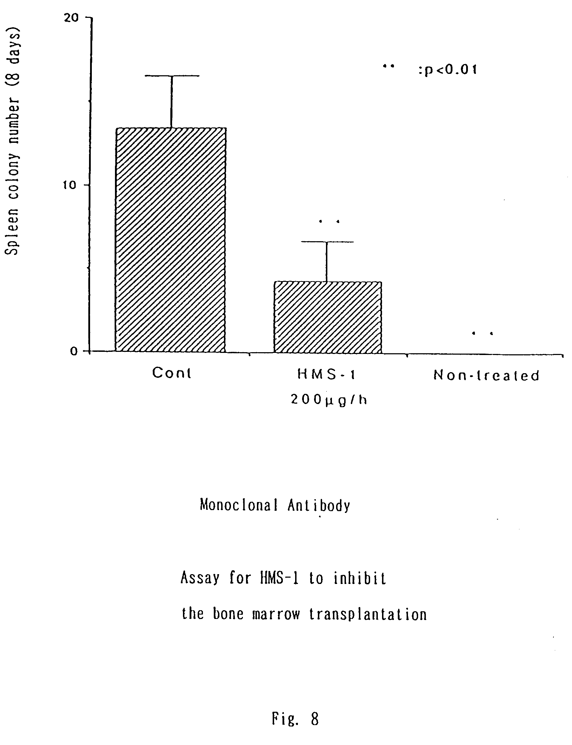

Next, an experiment upon the inhibition of the bone marrow transplantation was performed using these antibodies to examine characteristics thereof. The results are shown in Fig. 7 and Fig. 8. As is apparent from Fig. 7 and Fig. 8, while HMS-1 has an effect inhibiting the bone marrow transplantation, the effect was not found in GSPST-1. Namely, the above results were obtained by administering 1.0 × 10⁵/head of bone marrow cells and monoclonal antibodies to C57BL/6J mice, irradiated with a fatal dose of radiation (900 cGy), through the veins of tails, and observing the formation of splenic colonies. Incidentally, "Non-treated" in Fig. 8 shows the case with no administration of bone marrow cells. Accordingly, since the monoclonal antibody HMS-1 of the present invention is recognized to show the effect inhibiting the bone marrow transplantation according to the above experiment, it is apparent that the antibody inhibits also the homing of hematopoietic stem cells and the formation of CFU-S colonies.

Incidentally, it is obvious to those skilled in the art that effect of the monoclonal antibody to inhibit the bone marrow transplantation corresponds to the activities to inhibit the homing of hematopoietic stem cells and the forming of CFU-S colonies. For example, the monoclonal antibody (ACK-2 antibody, one of C-kit antibodies) established by using a known mast cell as an antigen is reported to have a function inhibiting the bone marrow transplantation in a mouse similarly to the monoclonal antibodies of the present invention [Blood, 78, 1706 (1991)] ; in the report, it is concluded according to the results of the experiment upon the inhibition of the bone marrow transplantation that the antibody inhibits the homing of hematopoietic stem cells and the forming of CFU-S colonies.

The above-mentioned known antibody ACK-2 is prepared by using a mast cell as an antigen, and the mast cell is a hematopoietic cell differentiated and proliferated from a hematopoietic stem cell. On the other hand, a stromal cell of hematopoietic tissue used in the present invention is not a hematopoietic cell but a cell supporting the differentiation and proliferation of a hematopoietic cell, and hence both are distinctly different.

Next, when a hybridoma producing HMS-1 was administered to the abdominal cavity of a nude mouse, it was revealed that the mouse did not die from storage of ascite. Hence, it is thought that the effect of HMS-1 inhibiting the bone marrow transplantation is not a phenomenon due to so-called apoptosis [it is also called self-destruction of cells, phenomenon that a nuclear chromatin DNA is digested at a nucleosome unit (so-called ladder formation) to result in the death of cells] but a phenomenon caused by the binding of a monoclonal antibody to a molecule for the homing of hematopoietic stem cells on the hematopoietic tissue. Hence, it is apparent that an antigen recognized by HMS-1 is a substance important for the homing of hematopoietic stem cells on the hematopoietic tissue.

As recognized in the experiment above, the monoclonal antibody HMS-1 of the present invention recognizes the surface antigen of a splenic stromal cell having potency supportive of the homing of hematopoietic stem cells and has a characteristic of inhibiting the homing of hematopoietic stem cells.

Thus, HMS-1 has a potency of inhibiting the homing of bone marrow cells on the hematopoietic tissue; it is reported that leukemic cells are released from the bone marrow according to the decrease of the expression of VLA4 and VLA5 of adhesion molecules of hematopoietic cells already reported [VLA molecule expression may be involved in the release of acute myeloid leukaemic cells from the bone marrow, Leuk Res., 16 (5), 469-474 (1992) ] , and it is thought that the monoclonal antibody to VLA4 and VLA5 improves the effect of remedies for leukemia by releasing leukemic cells from the bone marrow. Hence, the above HMS-1 may be used similarly in advance for the treatment of leukemia to obtain the same effect.

The monoclonal antibodies of the present invention have been described specifically according to the Example as above; the monoclonal antibodies of the present invention include those exemplified as represented examples above, but they are not always restricted to them but include all monoclonal antibodies having the same characteristic and function prepared in the same manner.

### Industrial Applicability

Since the monoclonal antibodies of the present invention recognize a substance important for the homing of bone marrow cells on the hematopoietic tissue as an antigen and have a characteristic of inhibiting the homing of hematopoietic stem cells, it is useful as medicine with a function of enhancing the effect of an anticancer agent, for example, improving the effect of the anticancer agent for leukemia by releasing acute myeloid leukemic cells from the bone marrow according to the above characteristic.

## Claims

1. A monoclonal antibody inhibiting the homing of hematopoietic stem cells and recognizing the surface antigen of a splenic stromal cell having potency supportive of the homing of hematopoietic stem cells.

2. A hybridoma producing the monoclonal antibody according to Claim 1.
